(19) Europäisches Patentamt
European Patent Office
Office européen des brevets

(11) Veröffentlichungsnummer: **0 414 018 A2**

# (12) EUROPÄISCHE PATENTANMELDUNG

(21) Anmeldenummer: **90115020.1**

(22) Anmeldetag: **04.08.90**

(51) Int. Cl.5: **C07D 491/08**, C07D 207/273, //(C07D491/08,307:00,209:00)

(30) Priorität: **19.08.89 DE 3927367**

(43) Veröffentlichungstag der Anmeldung: **27.02.91 Patentblatt 91/09**

(84) Benannte Vertragsstaaten: **AT BE CH DE DK ES FR GB GR IT LI LU NL SE**

(71) Anmelder: **BAYER AG**

**D-5090 Leverkusen 1 Bayerwerk(DE)**

Anmelder: **CHINESE ACADEMY OF MEDICAL SCIENCES**
**1 Xian Nong Tan Street**
**Beijing 100050(CN)**

(72) Erfinder: **Huang, Liang, Prof., Institute of Materia Medica**
**Chinese Academy of Medical Sciences**
**1 Xian Nong Tan Street, Beijing 100050(CN)**
Erfinder: **Liu, Gen-tao, Prof., Institute of Materia Medica**
**Chinese Academy of Medical Sciences**
**1 Xian Nong Tan Street, Beijing 100050(CN)**

(74) Vertreter: **Dänner, Klaus, Dr. et al**
**c/o Bayer AG Konzernverwaltung RP Patentabteilung**
**D-5090 Leverkusen 1 Bayerwerk(DE)**

## (54) Verfahren zur Herstellung von Dehydrocycloclausenamid und dessen Derivate in der racemischen Form und als optisch aktive (+)-oder (-)-Enantiomere.

(57) Die Erfindung betrifft ein Verfahren zur Herstellung von Dehydrocycloclausenamid der allgemeinen Formel (I)

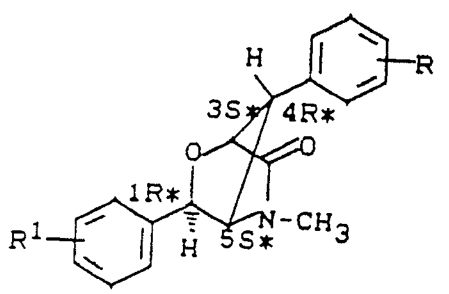

(I)

in welcher
R und $R^1$ die in der Beschreibung angegebene Bedeutung haben und dessen Derivate in der racemischen Form und als optisch aktive (+) oder (-)-Enantiomere.

EP 0 414 018 A2

# VERFAHREN ZUR HERSTELLUNG VON DEHYDROCYCLOCLAUSENAMID UND DESSEN DERIVATE IN DER RACEMISCHEN FORM UND ALS OPTISCH AKTIVE ( + ) ODER (-)-ENANTIOMERE

Die Erfindung betrifft ein Verfahren zur Herstellung von Dehydrocycloclausenamid und dessen Derivate in der racemischen Form und als optisch aktive ( + ) oder (-)-Enantiomere.

Es ist bereits bekannt, daß Dehydrocycloclausenamid aus dem wäßrigen Extrakt aus Blättern von Clausena Lansium (low) skeels gewonnen und seine Struktur durch chemische Reaktionen und durch Röntgenstrukturanalyse aufgeklärt wurde [vgl. DE-A1 34 31 257, Phytochemistry, 27 (2), 445-450]. Ferner ist bekannt, daß Dehydrocycloclausenamid den durch Tetrachlorkohlenstoff induzierten erhöhten Serumspiegel an Glutamin-pyruvat-Transaminase unterdrückt.

Da für pharmakologische Studien größere Mengen benötigt werden, andererseits aber durch das aufwendige Extraktionsverfahren aus 14 g getrockneten Blättern nur 1,5 g Dehydrocycloclausenamid gewonnen werden, war es notwendig ein Verfahren zur chemischen Synthese und Derivatisierung zur Verfügung zu stellen.

Es ist als überraschend zu bezeichnen, daß mit Hilfe des erfindungsgemäßen Verfahrens die erfindungsgemäße Verbindung der allgemeinen Formel (I)

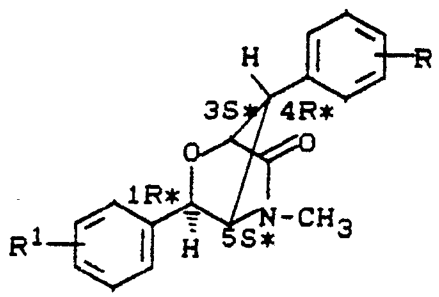

(I)

in welcher

R und $R^1$ gleich oder verschieden sind und für Wasserstoff oder Halogen stehen,

sowohl in der Racemform als auch in Form ihrer reinen Enantiomere hergestellt werden kann.

Das Produkt (Ia)

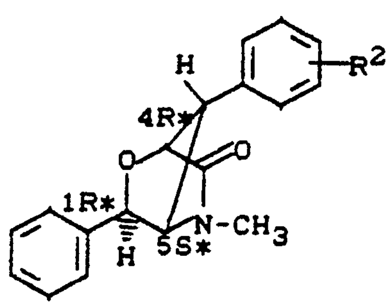

(Ia)

in welcher

$R^2$ für Wasserstoff steht

ist mit dem aus dem Pflanzenextrakt gewonnenen Dehydrocycloclausenamid identisch. Mit dem neuen Verfahren können im Vergleich zum Extraktionsverfahren größere Mengen in kürzerer Zeit und mit weniger Aufwand zur Verfügung gestellt werden und der Zugang zu neuen Derivaten ermöglicht werden.

Die Erfindung betrifft zunächst neue Verbindungen der allgemeinen Formel (Ib)

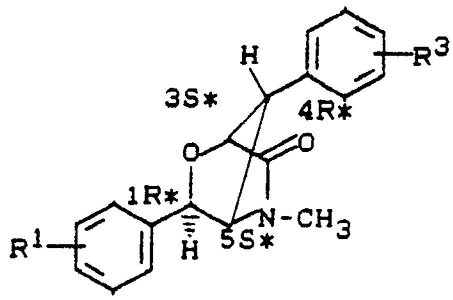

(Ib)

in welcher
$R^3$ - für Halogen steht und
$R^1$ die oben angegebene Bedeutung hat als Racemat und als (+)- oder (-)-Enantiomer.

Halogen steht bevorzugt für Fluor oder Chlor.

Die Erfindung betrifft weiterhin ein Verfahren zur Herstellung von Dehydrocycloclausenamid und seiner chemisch synthetisierbaren Derivate der allgemeinen Formel (I).

Die Verbindungen der allgemeinen Formel (I), als (+)-und (-)-Enantiomere oder als Racemat (±), können hergestellt werden, indem man

[A] Verbindungen der allgemeinen Formel (II)

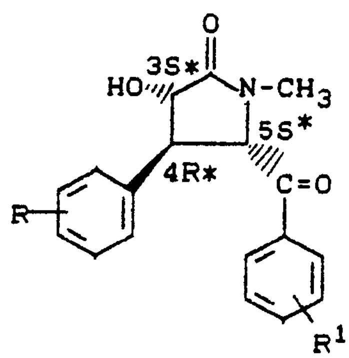

(±), (+), (-) (II)
(R,S-Nomenklatur wird beispielhaft an den Verbindungen der allgemeinen Formel (II) aufgeführt)

in welcher
R für Wasserstoff oder Halogen steht und
$R^1$ die oben angegebene Bedeutung hat
entweder als Racemat (±) oder als entsprechendes (+)-oder (-)-Enantiomer, zunächst mit 2,3-Dihydropyran zu Verbindungen der allgemeinen Formel (III)

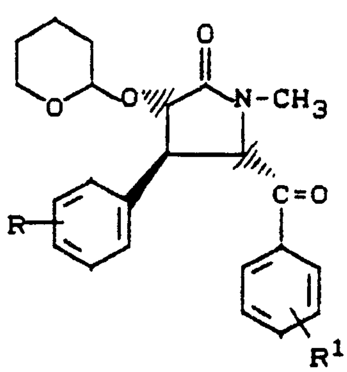

(±), (+), (-) (III)

in welcher
R und $R^1$ die oben angegebene Bedeutung haben,
verethert, dann nach üblicher Methode zu Verbindungen der allgemeinen Formel (IV)

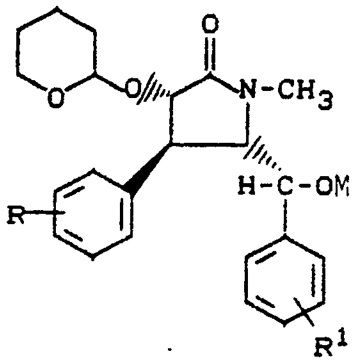

(±), (+), (-) (IV)

in welcher
R und $R^1$ die oben angegebene Bedeutung haben,
reduziert, dann in einem weiteren Schritt mit p-Methyltoluolsulfonsäurechlorid der Formel (V)

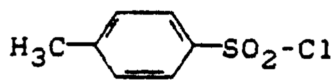

(V)

zu Verbindungen der allgemeinen Formel (VI)

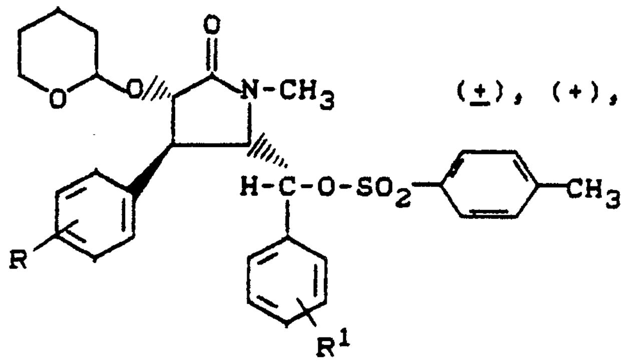

(±), (+), (-) (VI)

in welcher
M für ein Metall, bevorzugt ein Alkalimetall, Zink oder Aluminium steht;
R und $R^1$ die oben angegebene Bedeutung haben,
umsetzt, dann die Tetrahydropyranylschutzgruppe nach üblicher Methode abspaltet und in einem letzten Schritt mit 2,6-Lutidin der Formel (VII)

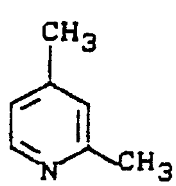

(VII)

umsetzt,
oder indem man
[B] Verbindungen der allgemeinen Formel (IVa)

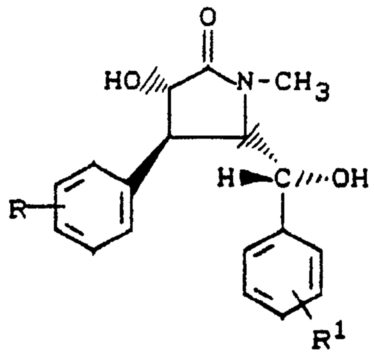

(±)

(IVa)

in welcher R und R¹ die oben angegebene Bedeutung haben, in der racemischen Form (±) mit Azodicarbonsäurediethylester der Formel (VIII),

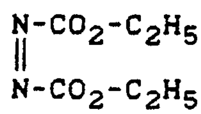

(VIII)

gegebenenfalls in Anwesenheit eines Hilfsstoffes, in inerten Lösemitteln umsetzt.

Die erfindungsgemäßen Verfahren können durch folgendes Formelschema erläutert werden:

[A]

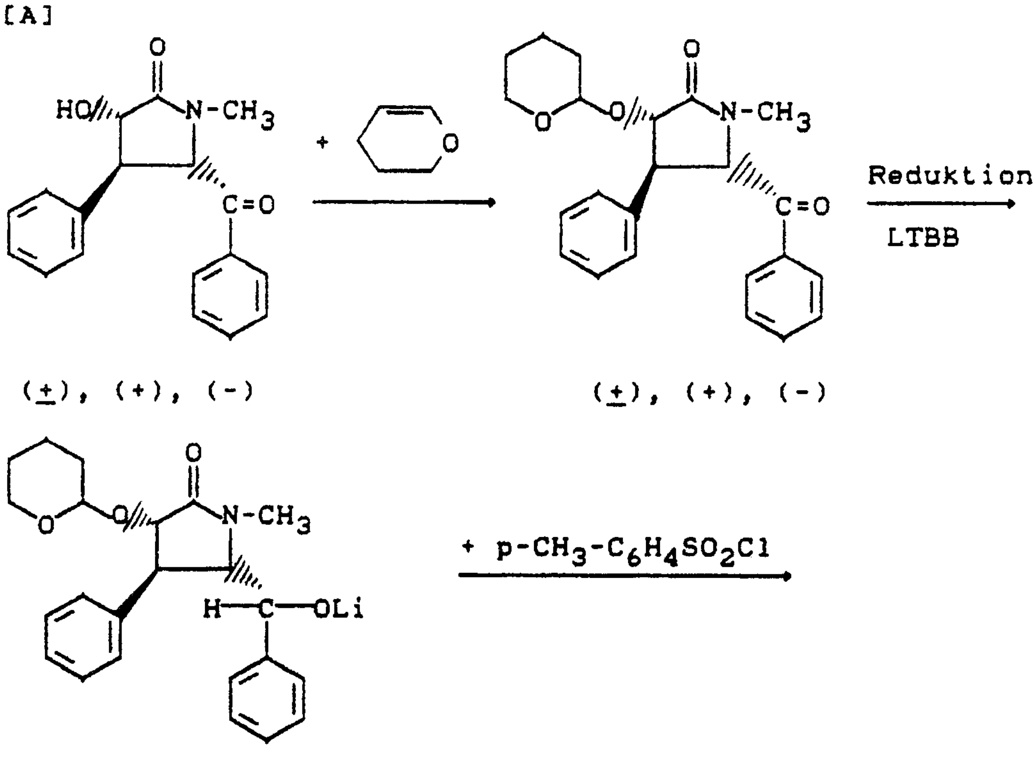


(±), (+), (-)      (±), (+), (-)

(±), (+), (-)

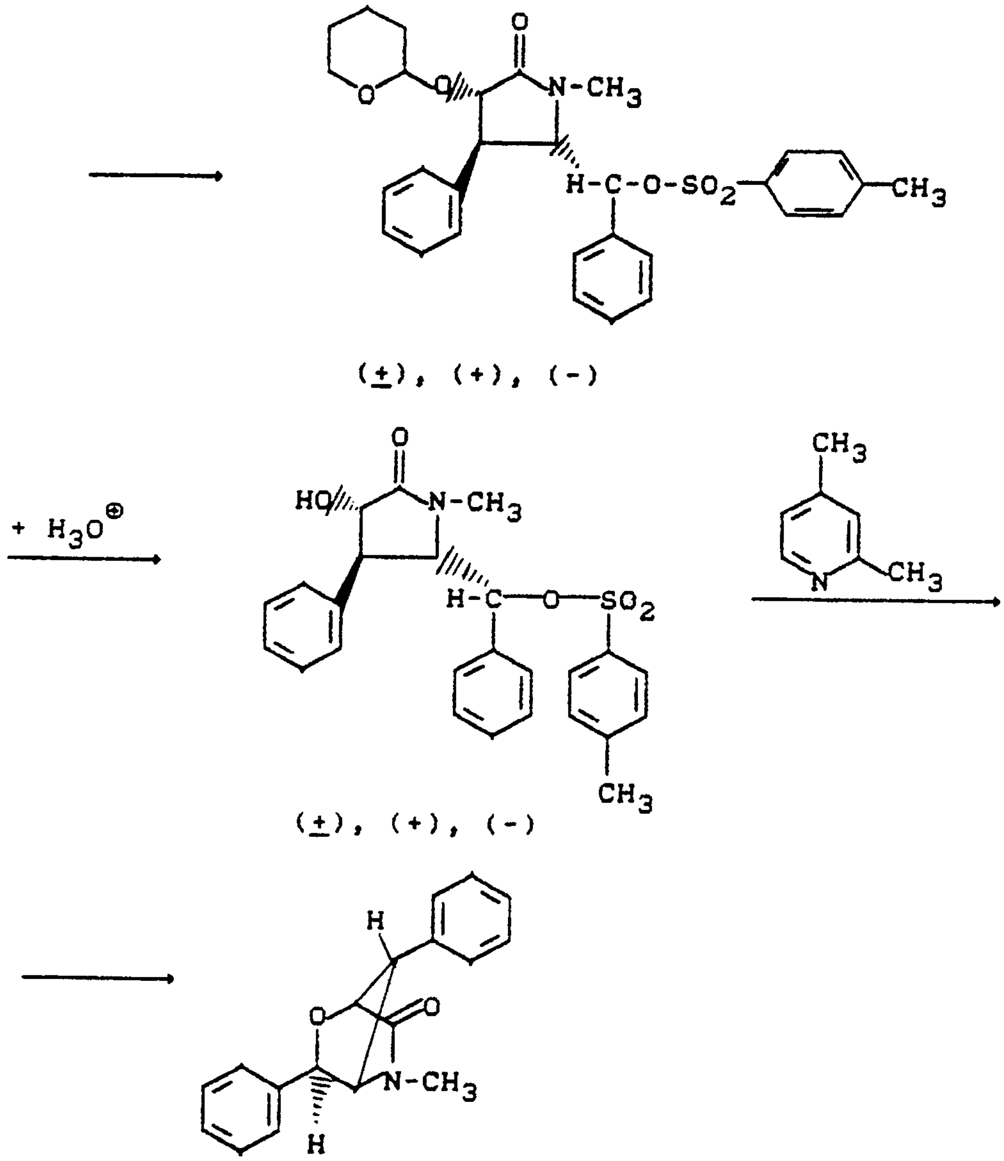


(±), (+), (-)

(±), (+), (-)

(±), (+), (-)

[B]

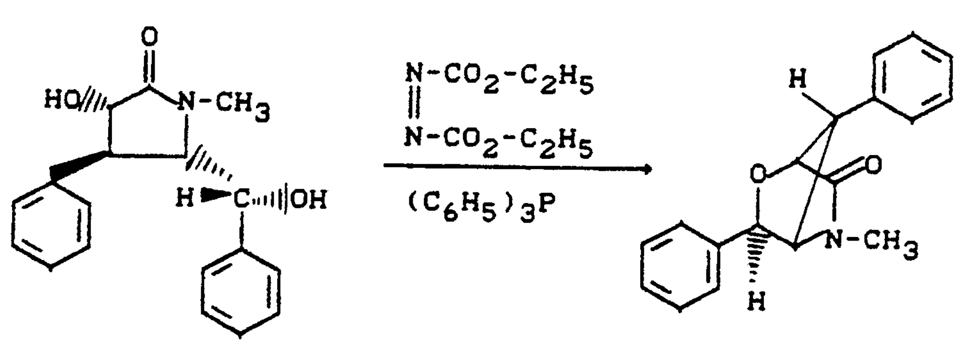

Als Lösemittel für die Veretherung mit 2,3-Dihydropyran eignen sich alle inerten organischen Lösemittel, die sich unter den Reaktionsbedingungen nicht verändern. Hierzu gehören bevorzugt Halogenkohlenwasserstoffe, wie beispielsweise Methylenchlorid oder Chloroform, oder Amide wie Hexamethylphosphorsäuretriamid oder Dimethylformamid. Besonders bevorzugt ist Methylenchlorid.

Die Veretherung verläuft in einem Temperaturbereich von -10° C bis +50° C, bevorzugt bei +30° C, bei Normaldruck.

Als Hilfsstoffe eignen sich gegebenenfalls Sulfonsäuren wie beispielsweise Methansulfonsäure, Ethansulfonsäure, Benzolsulfonsäure oder Toluolsulfonsäure, oder die entsprechenden Pyridiniumsalze wie beispielsweise Pyridinium-p-Toluolsulfonat. Bevorzugt ist Pyridinium-p-Toluolsulfonat.

Als Lösemittel für die Reduktion eignen sich alle inerten organischen Lösemittel, die sich unter den Reaktionsbedingungen nicht verändern. Hierzu gehören be vorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure. Ebenso ist es möglich, Gemische der genannten Lösemittel zu verwenden. Bevorzut sind Diethylether und Tetrahydrofuran.

Die Reduktion der Ketofunktion zur Hydroxyfunktion kann mit den üblichen Reduktionsmitteln durchgeführt werden. Besonders geeignet sind Metallhydride und komplexe Metallhydride wie beispielsweise Lithiumboranate, Natriumboranate, Lithiumaluminiumhydrid, Zinkborhydride oder Aluminiumtriisopropylat. Besonders bevorzugt sind Lithiumborhydrid, Natriumborhydrid, Zinkborhydrid und Aluminiumtriisopropylat.

Die Reduktion kann bei normalem, aber auch bei erhöhtem oder erniedrigtem Druck (z.B. 0,5 - 5 bar) durchgeführt werden. Im allgemeinen arbeitet man bei Normaldruck.

Die Umsetzung mit p-Tosylchlorid verläuft in einem der oben aufgeführten Lösemittel bei einer Reaktionstemperatur von -30° C bis 0° C bei Normaldruck, bevorzugt in Tetrahydrofuran bei einer Temperatur von -20° C bis -5° C bei Normaldruck.

Die Abspaltung der Schutzgruppe Tetrahydropyranyl erfolgt in an sich bekannter Weise, beispielsweise mit Protonensäuren. Hierzu gehören bevorzugt anorganische Säuren wie beispielsweise Salzsäure oder Schwefelsäure.

Die Reaktion mit 2,6-Lutidin (2,6-Dimethylpyridin) erfolgt ebenfalls in einem der oben aufgeführten inerten Lösemitteln, bevorzugt in Methylenchlorid, unter Schutzgasatmosphäre. Die Reaktionstemperatur liegt bei +90° C bis +170° C, bevorzugt bei +100° C bis +150° C. Es wird grundsätzlich bei Normaldruck gearbeitet.

Die Umsetzung mit Azodicarbonsäureester verläuft in einem der oben aufgeführten inerten Lösemitteln, bevorzugt in Tetrahydrofuran, in einem Temperaturbereich von -10° C bis +60° C, bevorzugt von 0° C bis +30° C. Die Reaktion kann sowohl bei Normaldruck, als auch bei erhöhtem oder erniedrigtem Druck durchgeführt werden. Bevorzugt wird bei Normaldruck gearbeitet.

Als Hilfsstoffe werden hierbei Trialkylphosphane wie beispielsweise Triphenylphosphan oder Ethyldiphenylphosphan, bevorzugt Triphenylphosphan, eingesetzt.

Die Verbindungen der allgemeinen Formel (II) sind als Stoffe an sich bekannt [vgl. DE-A1 34 31 257], können jetzt aber chemisch hergestellt werden, indem man Verbindungen der allgemeinen Formel (IX)

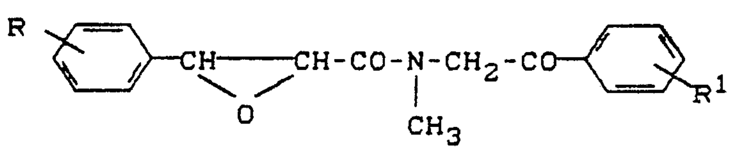

(IX)

in welcher

R und $R^1$ die oben angegebene Bedeutung haben,

in inerten Lösemitteln, gegebenenfalls in Anwesenheit einer Base cyclisiert und die entstehenden Isomere nach üblicher Methode chromatographisch trennt.

Als Lösemittel für die Cyclisierung eignen sich inerte organische Lösemittel wie Ether, beispielsweise Tetrahydrofuran, Diethylether oder Dioxan, oder Alkohole wie beispielsweise Methanol oder Ethanol, oder Halogenkohlenwasserstoffe wie beispielsweise Methylenchlorid oder Tetrachlorkohlenstoff, oder deren Gemische, gegebenenfalls auch mit Wasser. Bevorzugt sind Tetrahydrofuran, Methanol und Methylenchlorid.

Als Basen zur Cyclisierung eignen sich Alkalialkoholate, Alkaliamide oder Alkalihydride wie beispielsweise Natriumethanolat, Natriummethanolat, Kalium-, Natrium-oder Lithiumbutanolat, Lithium-, Natrium- oder

Kaliumhydroxid, Natriumhydrid, Lithiumdiisopropylamid, Butyllithium oder Ammoniumhydroxide wie beispielsweise Tetramethylammoniumhydroxid. Bevorzugt sind Lithiumdiisopropylamid, Natriummethanolat, Lithiumhydroxid oder Tetramethylammoniumhydroxid.

Die Reaktionstemperaturen liegen zwischen -70 °C und +40 °C. Bevorzugt wird zwischen -65 °C und +30 °C gearbeitet.

Die Cyclisierung kann bei normalem, aber auch bei erhöhtem oder erniedrigtem Druck durchgeführt werden. Im allgemeinen arbeitet man bei Normaldruck.

Die reinen (+)- oder (-)-Enantiomere der Verbindungen der allgemeinen Formel (II) können hergestellt werden, indem man ausgehend vom Racemat der Verbindungen der allgemeinen Formel (II)

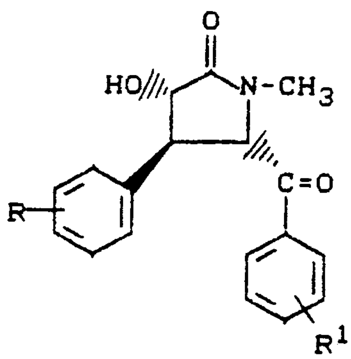

(±) (II)

in welcher
R und $R^1$ die oben angegebene Bedeutung haben,
durch die Umsetzung mit (-)-Menthoxyessigsäurechlorid der allgemeinen Formel (X)

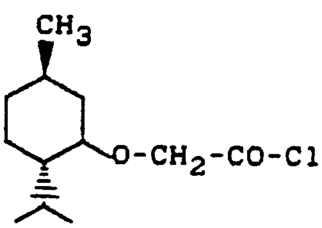

(X)

in einem der oben aufgeführten inerten Lösemitteln, vorzugsweise Methylenchlorid in Anwesenheit einer Base die entsprechenden Diastereomere herstellt und anschließend mit Säuren den Menthoxyessigsäurerest unter Freisetzung der Hydroxylfunktion abspaltet.

Das Verfahren kann durch folgendes Formelschema verdeutlicht werden:

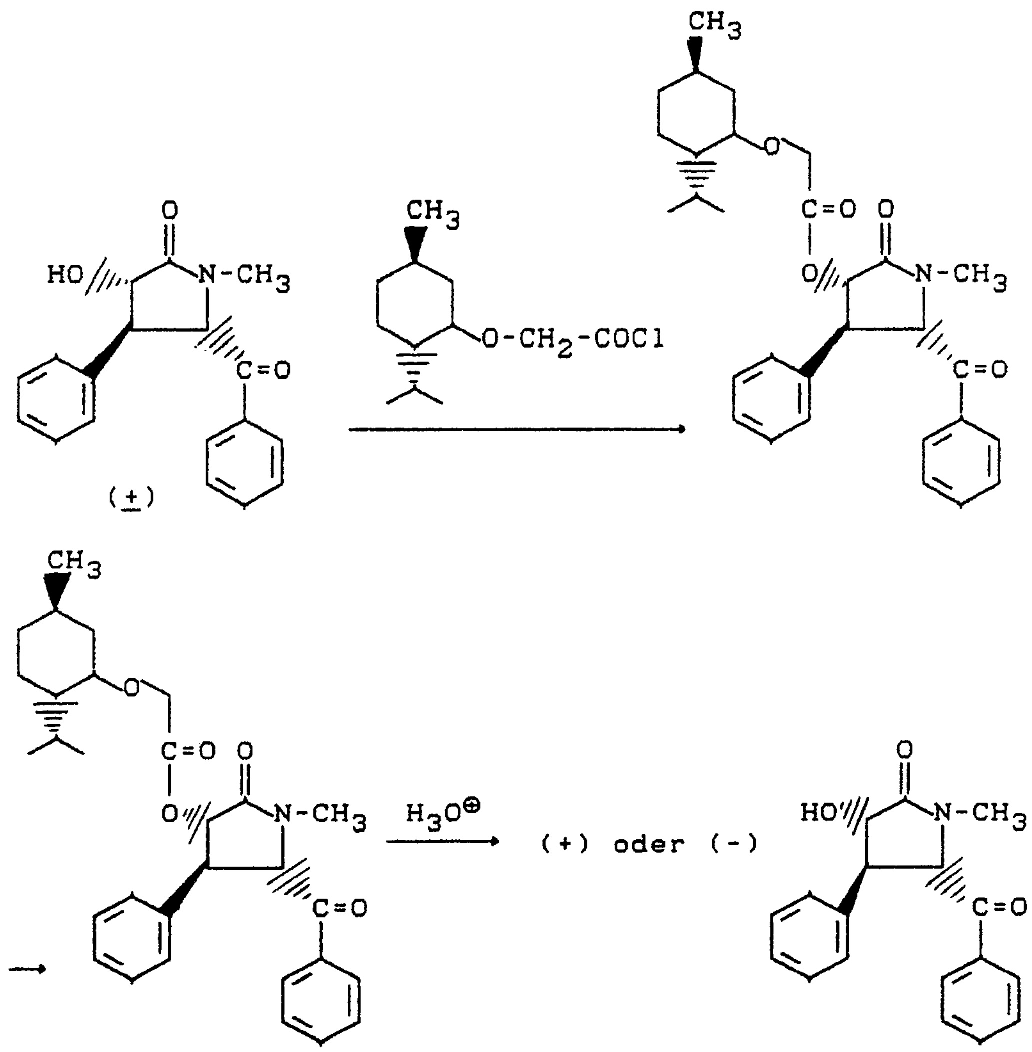


Die Verbindungen der allgemeinen Formel (IVa) sind an sich bekannt [vgl. DE-A1 34 31 257].

Als Basen eignen sich organische Amine wie Triethylamin, Picolin oder N-Methylpiperidin oder Pyridin. Bevorzugt ist Pyridin.

Die Umsetzung wird im allgemeinen in einem Temperaturbereich von 0 °C bis +60 °C, bevorzugt bei Raumtemperatur, durchgeführt.

Die Reaktion wird im allgemeinen bei Normaldruck durchgeführt. Es ist jedoch ebenso möglich, die Reaktion bei erhöhtem oder erniedrigtem Druck durchzuführen.

Zur Abspaltung der Hydroxyschutzgruppe werden im allgemeinen Sulfonsäuren mit $C_1$-$C_4$-Alkylresten oder Arylresten wie beispielsweise Methansulfonsäure, Ethansulfonsäure, Benzolsulfonsäure oder Toluolsulfonsäure verwendet. Bevorzugt ist Toluolsulfonsäure.

Die Reaktion wird in einem Temperaturbereich von +50 °C bis +150 °C, vorzugsweise bei +80 °C bis +130 °C und Normaldruck, durchgeführt.

Die Verbindung der Formel (V) ist bekannt [vgl. Beilstein 9, 498].

2,6-Lutidin der Formel (VII) ist ebenfalls bekannt [vgl. Beilstein 20, 244].

Die Verbindungen der allgemeinen Formel (IVa) sind bekannt oder können nach bekannter Methode hergestellt werden [vgl. DE-A1 35 37 075].

Azodicarbonsäurediethylester ist bekannt [vgl. Experimentia, 25, 680 (1969); Tetrahedron 26, 5731 (1970)].

(-)-Menthoxyessigsäurechlorid der Formel (IX) ist bekannt und kann aus (-)-Menthoxyessigsäure unter Einwirkung von Chlorierungsmitteln wie beispielsweise Thionylchlorid hergestellt werden [vgl. Beilstein, 6 (1), 25].

Die Verbindungen der allgemeinen Formel (Ib) haben eine ausgeprägte vor cerebraler Hypoxie schützende und antiamnesische Wirkung. Sie können deshalb als cerebrale Therapeutika und Nootropika

eingesetzt werden. Außerdem bewirken die erfindungsgemäßen Verbindungen der allgemeinen Formel (Ib) eine Senkung der erhöhten Glutaminpyruvat-Transaminase im Serum, bei einer Zellschädigung. Sie können deshalb als Arzneimittel bei Herzinfarkt, akuter Hepatitis, chronischer Hepatitis, Pankreatitis oder bei Muskelerkrankungen eingesetzt werden.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Unter nichttoxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können neben dem oder den Wirkstoffen die üblichen Trägerstoffe enthalten, wie (a) Füll- und Streckmittel, z.B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffen enthalten, z.B. Polyethylenglkole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe Sprays können zusätzlich die üblichen Treibmittel, z.B. Chlorfluorkohlenwasserstoffe enthalten.

Lösungen und Emulsionen können neben dem oder den Wirkstoffen die üblichen Trägerstoffe wie Lösungsmittel, Lö sungsvermittler und Emulgatoren, z.B. Wasser, Ethylalkohol, Isopropylalkohol, Ethylcarbonat, Ethylacetat, Benzylalkohol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazetischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindung

Beispiel I

(-)-Menthoxyessigsäurechlorid

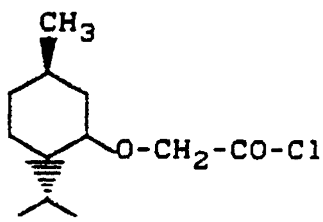

0,771 g (3,6 mmol) (-)-Menthoxyessigsäure werden mit 2,1 g Thionylchlorid 4 bis 5 h unter Rückfluß gerührt. Aus der Lösung wird zunächst das Thionylchlorid unter vermindertem Druck abdestilliert. Anschließend wird mit 3 ml trockenem Benzol versetzt, um restliches Thionylchlorid durch Vakuumdestillation zu entfernen. Man erhält 0,9 g der Titelverbindung.

Herstellungsbeispiele

Beispiel 1

N-methyl-N-phenacyl-3-phenylglycidamid

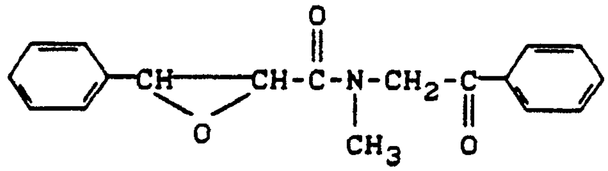

a) 2,4 g (0,0086 mol) N-Methyl-N-phenacylzimtamid und 8,6 g (0,04 mol) m-Chlorperbenzoesäure werden in 170 ml Chloroform gelöst und 2 Tage bei Raumtemperatur stehen gelassen. Der Niederschlag wird abfiltriert und das Filtrat wird nacheinander mit Natriumsulfitlösung (10%), Natriumcarbonatlösung

(10%) und Wasser gewaschen und über wasserfreiem Natriumsulfat getrocknet. Das Lösemittel wird entfernt, der Rückstand in 6 ml Benzol/trockenem Ether (1:1) gelöst und im Eisschrank gekühlt. Nach Abtrennen von nicht umgesetztem Ausgangsmaterial wird abfiltriert. Der ölige Rückstand wird chromatographisch gereinigt.

Ausbeute: 1,25 g (49,2% der Theorie)

$^1$H-NMR (90 MHz, $CDCl_3$): $\delta$ = 3,09 (s) + 3,21 (s, 3H, $NCH_3$); 3,49 (d) + 3,88 (d, J = 2,7 Hz, 1H); 4,04 (d) + 4,15 (d, J = 2,7 Hz, 1H); 4,82, 5,04 (AB, J = 18 Hz) + 4,96 (s, 2H); 7,30 - 7,8 (m, 8H); 8,01 (dd, J = 1,8 Hz, 8 Hz) + 8,06 (dd, J = 2,7 Hz, 8 Hz, 2H).

b) Zu einer Lösung von 5,94 g (0,02 mol) N-Methyl-N-($\beta$-hydroxy-$\beta$-phenyl)ethyl-3-phenylglycidamid werden 36 g aktiviertes Mangandioxid unter kräftigem Rühren zugegeben. Es wird 1,5 h gerührt bis bei der dünnschichtchromatographischen Überprüfung kein Ausgangsmaterial mehr nachweisbar ist. Es wird vom Mangandioxid abfiltriert und mit Methylenchlorid gewaschen. Die vereinigten Filtrate werden zunächst mit 20 ml einer 15% Natriumhydrogensulfit-Lösung und 20 ml einer gesättigten Natriumchlorid-Lösung gewaschen und über wasserfreiem Natriumsulfat getrocknet. Man erhält einen viskosen transparenten Rückstand, der sich beim Stehenlassen verfestigt. Durch Umkristallisieren erhält man weiße Nadeln.

Ausbeute: 4,42 g (75% der Theorie)

F°C: 76,5 - 79°C

Nach einer zweiten Umkristallisation wird ein Schmelzpunkt von 78,5 - 80°C festgestellt.

$^1$H-NMR ($CDCl_3$): $\delta$ = 3,12 (s) + 3,26 (s); (3H, $NCH_3$); 3,52 (d) + 3,85 (d, 1H, J = 2 Hz); 4,07 (d), 4,19 (d, 1H, J = 2 Hz); 4,88 (d); 5,10 (d); (2H, J = 18 Hz, $PhCOCH_2$); 7,30 - 8,16 (m, 10H).

Beispiel 2

(±) 3(S*), 4(R*), 5(S*)-1-methyl-3-hydroxy-4-phenyl-5-benzoyl-pyrrolidin-2-on

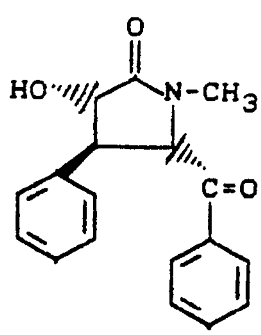

Zu einer Lösung von 4 mmol Lithiumdiisopropylamid in 5 ml Tetrahydrofuran werden bei -72°C 490 mg (1,66 mmol) der Verbindung aus Beispiel 1 in 10 ml Tetrahydrofuran unter Rühren und Kühlen zugetropft. Es wird über 4 h gekühlt und gerührt, bis kein Ausgangsmaterial mehr nachweisbar ist. Dann werden 100 ml Wasser langsam zugegeben. Tetrahydrofuran wird im Vakuum entfernt. Man erhält 680 mg eines braunen halbfesten Rohproduktes, das chromatographisch (Chromatotron) gereinigt wird Summenformel: $C_{18}H_{17}NO_3$

| | | | | |
|---|---|---|---|---|
| Elementaranalyse: | kalk.: | C 73,22 | H 5,76 | N 4,75 |
| | gef. : | C 73,27 | H 5,67 | N 4,70 |

$^1$H-NMR ($CDCl_3$): $\delta$ = 2,92 (s, 3H, $NCH_3$); 3,92 (t, J = 8,5 Hz, 1H, $C_4$H); 3,40 (br, s, 1H, austauschbar mit $D_2O$); 4,93 (d, J = 8,5 Hz, 1H, $C_3$-H); 5,50 (d, J = 8,5 Hz, 1H, $C_5$-H); 7,04-7,84 (m, 10H, ArH).

Beispiel 3a,b

(+)-3(S*), 4(R*), 5(S*)-1-methyl-3-menthoxyessigsäure-4-phenyl-5-benzoyl-pyrrolidin-2-on (Beispiel 3a)

(-)-3(S*), 4(R*), 5(S*)-1-methyl-3-menthoxyessigsäure-4-phenyl-5-benzoyl-pyrrolidin-2-on (Beispiel 3b)

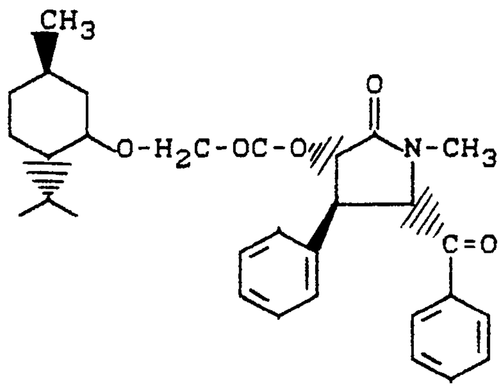

0,9 g der Verbindung aus Beispiel I werden in 10 ml Methylenchlorid gelöst und anschließend mit 0,88 g (3 mmol) Racemat der Verbindung aus Beispiel 2 versetzt. Nach dem Lösen wird die Reaktionsmischung im Eisbad gekühlt und mit 0,5 ml Pyridin versetzt. Die Reaktionslösung wird 4 h bei Raumtemperatur gerührt. Dünnschichtchromatographische Untersuchungen ($SiO_2$-Platte) ergeben zwei Flecken mit den $R_f$-Werten 0,37 und 0,42 mit Laufmittelsystem Ether/Hexan (2:1). Die Reaktionslösung wird mit 10 ml Methylenchlorid verdünnt und nacheinander durch Waschen mit 20 ml 2 N Salzsäure, gesättigter Natriumcarbonatlösung und gesättigter Natriumchloridlösung neutralisiert. Nach dem Trocknen über Natriumsulfat wird das Lösemittel entfernt und 1,69 g eines viskosen Feststoffs erhalten, der in 200 ml Hexan aufgenommen wird und durch Ultraschall in ein feines Pulver überführt wird. Man erhält 0,747 g eines Feststoffs. Nach Umkristallisation aus Methanol erhält man 0,48 g (33% der Theorie) des einen Diastereomers (3a) mit dem Schmelzpunkt 171-172,5 °C, $R_f$ = 0,4.
$[\alpha]_D^{15}$ = 52,6 (C = 0,95 Chloroform)
$^1$H-NMR ($CDCl_3$, 90 MHz): $\delta$ = 0,64 - 2,40 (m, 18H, Menthol H); 2,98 (s, 3H, N-$CH_3$); 3,1 (m, 1H); 3,32 (t, 1H, J = 4,7 Hz, $C_4$-H); 4,09 (s, 2H, -O-$CH_2$-C=O); 5,04 (d, 1H, J = 4,7 Hz, $C_5$-H); 5,40 (d, 1H, J = 4,7 Hz, $C_3$-H); 7,10 - 7,70 (m, 10H, ArH).
Die Hexanlösung wird eingeengt, und es liegen 0,81 g eines öligen Rückstandes vor. Dieser wird auf einer mit 60 g $SiO_2$ beschichteten Säule chromatographiert. 0,6 g (41% der Theorie) des anderen Diastereomers (3b) mit $R_f$ = 0,37 werden erhalten. Nach Umkristallisation aus Hexan zeigt der Feststoff einen Schmelzpunkt von 105-106,5 °C.
$[\alpha]_D^{18}$ = -32,9 (c = 1,1 Chloroform)
$^1$H-NMR ($CDCl_3$, 90 MHz): $\delta$ = 0,64 - 2,40 (m, 18H, menthol-H); 2,98 (s, 3H, $NCH_3$); 3,0 - 3,2 (m, 1H); 3,34 (t, 1H, J = 5,0 Hz, $C_4$-H); 4,11 (s, 2H, -$OCH_2$-C=O); 5,06 (d, 1H, J = 5 Hz, $C_5$-H); 5,46 (d, 1H, J = 5 Hz, $C_3$-H); 7,00 - 7,70 (m, 10H, ArH).

Beispiel 4a,b

(+)-1-methyl-3-hydroxy-4-phenyl-5-benzoyl-pyrrolidin-2-on (Beispiel 4a)

(-)-1-methyl-3-hydroxy-4-phenyl-5-benzoyl-pyrrolidin-2-on (Beispiel 4b)

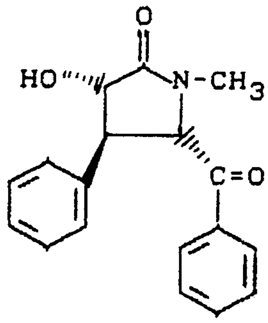

a) 404 mg (0,82 mmol) des einen Diastereomers aus Beispiel 3a werden in 40 ml Methanol und 10 ml Wasser 7 h unter Rückfluß mit 190 mg p-Toluolsulfonsäure gekocht. Das Lösemittel wird anschließend entfernt. Der verbleibende Rückstand wird in 30 ml Methylenchlorid gelöst. Das Lösemittel wird mit gesättigter Natriumbicarbonatlösung und Natriumchloridlösung gewaschen und über wasserfreiem Natriumsulfat getrocknet. Nach Entfernen des Lösemittels erhält man einen Feststoff, der aus dem System Ethylacetat/Hexan (5:2) kristallisiert.
Ausbeute: 210 mg (86% der Theorie), weiße Nadeln F°C: 166-169°C
$[\alpha]_D^{15}$ = -14,55 (C = 0,54 in Chloroform)
$^1$H-NMR ($CDCl_3$, 90 MHz): δ = 2,40 (br, 1H, OH); 2,93 (s, 3H, $NCH_3$); 3,27 (t, 1H, J = 7,0 Hz, $C_4$-H); 4,46 (d, 1H, J = 7,0 Hz, $C_3$-H); 5,05 (d, 1H; J = 7,0 Hz, $C_5$-H); 7,10 - 7,70 (m, 10H, ArH).
b) 442 mg des anderen Diastereomers aus Beispiel 3b werden in Analogie zu der unter a) aufgeführten Vorschrift hydrolysiert. Man erhält 217 mg eines weißen Feststoffs, der aus dem System Ethylacetat/Cyclohexan kristallisiert.
Ausbeute: 217 mg (82% der Theorie)
F°C: 170-172°C
$[\alpha]_D^{15}$ = + 15,3 (C = 0,47 in Chloroform)
$^1$H-NMR ($CDCl_3$, 90 MHz): δ = 2,74 (br, 1H, OH); 2,92 (s, 3H, $NCH_3$); 3,27 (t, 1H, J = 7,0 Hz, $C_4$-H); 4,46 (d, 1H, J = 7,0 Hz, $C_3$-H); 5,05 (d, 1H, J = 7,0 Hz, $C_5$-H); 7,10 - 7,70 (m, 10H, ArH).

Beispiel 5a,b,c

(±) 3(S*), 4(R*), 5(S*)-1-methyl-3-tetrahydropyranyloxy-4-phenyl-5-benzoyl-pyrrolidin-2-on (Beispiel 5a)

(+) 3(S*), 4(R*), 5(S*)-1-methyl-3-tetrahydropyranyloxy-4-phenyl-5-benzoyl-pyrrolidin-2-on (Beispiel 5b)

(-) 3(S*), 4(R*), 5(S*)-1-methyl-3-tetrahydropyranyloxy-4-phenyl-5-benzoyl-pyrrolidin-2-on (Beispiel 5c)

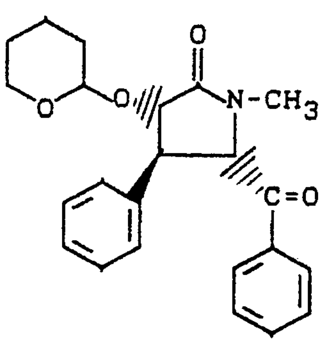

0,89 g (3 mmol) der Verbindung aus Beispiel 2, 4a oder 4b werden in 5 ml Methylenchlorid gelöst und

anschließend mit 760 mg 2,3-Dihydropyran und 75 mg Pyridinium-p-Toluolsulfonat versetzt. Die Reaktionslösung wird über Nacht bei Raumtemperatur gerührt, anschließend wird dünnschichtchromatographisch kontrolliert, ob die Ausgangsverbindung vollständig umgesetzt ist. Es werden 20 ml Methylenchlorid zugesetzt und die Lösung mit Natriumsulfatlösung gewaschen. Die Lösung wird über wasserfreiem Magnesiumsulfat getrocknet. Nach Einengen der organischen Phase erhält man einen weißen Feststoff. Es wird über eine Kieselgelsäule chromatographiert und der Feststoff aus Methylenchlorid/Ether umkristallisiert. Man erhält 1,05 g (92% der Theorie) der Titelverbindung.

F°C: 173-175°C

$^1$H-NMR ($CDCl_3$, 90 MHz): $\delta$ = 1,2 - 2,0 (m, 6H) 2,93 (s, 3H, $NCH_3$); 3,10 - 3,48 (m, 3H, $C_4$-H); 4,46 + 4,64 (d+d, 1H, J = 6,3 Hz, $C_3$-H); 4,97 + 4,95 (d+d, 1H, J = 5,5 Hz, $C_5$-H); 5,17 + 4,10 (m+m, 1H); 7,10 - 7,70 (m, 10H, ArH).

Die oben aufgeführte Vorschrift ermöglicht die Darstellung der Titelverbindungen aus dem optisch aktiven 3(S*), 4(R*), 5(S*)-1-methyl-3-hydroxy-5-benzoyl-4-phenyl-pyrrolidin-2-on

a) Aus 0,443 g des (-)-Isomers (5c) erhält man 0,51 g (90% der Theorie) des entsprechenden Ethers mit dem Schmelzpunkt 181-187°C.

$[\alpha]_D^{15}$ = +13,5 (C = 0,42 in Chloroform)

Epimerverhältnis: 2:1

b) Das (+)-Isomer (5b) wird in einer Menge von 426 mg (91% der Theorie) erhalten.

F°C: 194-200°C

$[\alpha]_D^{15}$ = -11,9 (C = 0,024 in Chloroform)

Epimerverhältnis 1:1

Beispiel 6a,b,c

(±) 3 (S*), 4(R*), 5(S*)-1-methyl-3-tetrahydropyranolyloxy-4-p-chlorophenyl-5-benzoyl-pyrrolidin-2-on (6a)

(+) 3 (S*), 4(R*), 5(S*)-1-methyl-3-tetrahydropyranolyloxy-4-p-chlorophenyl-5-benzoyl-pyrrolidin-2-on (6b)

(-) 3 (S*), 4(R*), 5(S*)-1-methyl-3-tetrahydropyranolyloxy-4-p-chlorophenyl-5-benzoyl-pyrrolidin-2-on (6c)

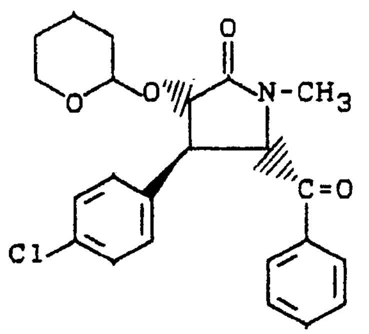

Zu einer Lösung von 1,65 g (5,0 mmol) (±) 3(S*), 4(R*), 5(S*)-1-methyl-3-hydroxy-4-p-chlorophenyl-5-benzoyl-pyrrolidin-2-on in 50 ml trockenem Methylenchlorid werden 125 mg (0,5 mmol) Pyridinium-p-toluolsulfonat und 15 ml (17 mmol) 2,3-Dihydropyran gegeben. Die Reaktionslösung wird 7 h bei Raumtemperatur gerührt und in Analogie zur Vorschrift des Beispiels 5 aufgearbeitet.

Man erhält zunächst 1,9 g weiße Kristalle vom Schmelzpunkt 123-125°C mit dem $R_F$-Wert 0,71 im Laufmittelsystem Ethylacetat/Hexan (2:1). Es wird aus Tetrahydrofuran und Isopropylether umkristallisiert.

Fp°C: 129-132°C

Beispiel 7a,b,c

(±) 3 (S*), 4(R*), 5(S*)-1-methyl-3-tetrahydropyranolyloxy-4-p-fluorophenyl-5-benzoyl-pyrrolidin-2-on (7a)

(+) 3 (S*), 4(R*), 5(S*)-1-methyl-3-tetrahydropyranolyloxy-4-p-fluorophenyl-5-benzoyl-pyrrolidin-2-on (7b)

(-) 3 (S*), 4(R*), 5(S*)-1-methyl-3-tetrahydropyranolyloxy-4-p-fluorophenyl-5-benzoyl-pyrrolidin-2-on (7c)

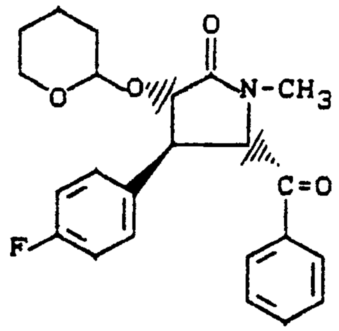

Die Titelverbindung wird in Analogie zur Vorschrift des Beispiels 6 hergestellt.
Ausbeute: 1,84 g (93% der Theorie)
Fp° C: 159 - 165° C
Epimerverhältnis: 2:1

Beispiel 8a,b,c

3 (S*), 4(R*), 5(S*)-1-methyl-3-hydroxy-5-tosyloxybenzylpyrrolidin-2-on

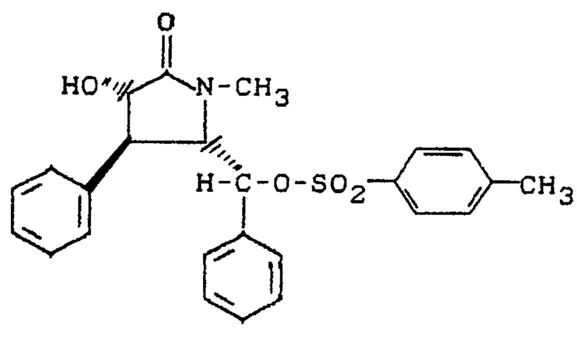

Eine Lösung von 0,190 g (0,5 mmol) der Verbindung aus Beispiel 7 in 5 ml Tetrahydrofuran wird auf -10° C gekühlt und 1,5 ml einer 1 M Tetrahydrofuranlösung mit Lithiumtri-sec-butyl-borhydrid (1,5 mmol) unter Stickstoffatmosphäre zugegeben. Es wird bei -10° C 1 h gerührt, anschließend 190 mg (1,0 mmol) p-Chlor-toluolsulfonsäure in 2 ml Tetrahydrofuran zugefügt und über Nacht stehen gelassen. Die Reaktionslösung wird 3 mal mit je 10 ml Methylenchlorid extrahiert. Die Methylenchloridlösung wird durch Waschen mit 2 N Natriumhydroxid und Natriumchloridlösung neutralisiert. Nach Trocknung über wasserfreiem Natriumsulfat wird vom Rückstand abfiltriert und zur Trockene eingeengt.

Der Rückstand wird in wasserfreiem Ethanol aufgenommen und auf 60° C erhitzt; anschließend werden 20 g p-Toluolsulfonsäure innerhalb von 10 min zugegeben und auf -15° C gekühlt. Man erhält weiße Nadeln vom Schmelzpunkt 171-172° C.

$^1$H-NMR ($CDCl_3$, 90 MHz): $\delta$ = 2,20 (1H, OH); 2,36 (s, 3H, Ar-$CH_3$); 2,84 (s, 3H, $NCH_3$); 3,15 (t, 1H, J = 5,5

Hz, $C_4$-H); 3,90 (dd, 1H, J = 4,0 Hz, 5,5 Hz, $C_5$-H); 4,14 (d, J = 5,5 Hz, 1H, $C_3$-H); 5,69 (d, 1H, J = 4 Hz, $C_7$-H); 6,80 -6,95 (m, 2H, ArH); 7,0 - 7,25 (m, 10H, ArH); 7,56 (d, 2H, J = 7,5 Hz, ArH).

Bei der oben aufgeführten Vorschrift wird das optisch aktive 3 (S*), 4(R*), 5 (S*)-1-methyl-3-tetrahydropyranyloxy-4-phenyl-5-benzoyl-pyrrolidin-2-on eingesetzt.

Ausgehend von 379 mg (1,0 mmol) des (-)-Enantiomeres (7c) erhält man 381 mg (84,3% der Theorie) des weißen kristallinen optisch aktiven (+) 3 (S*), 4 (R*), 5 (S*), 7(R*)-1-methyl-3-hydroxy-4-phenyl-5-(α-tosyloxy-benzyl)-pyrrolidin-2-on (8b).

Fp° C: 157,5 - 158,5° C

$[\alpha]_D^{15}$ = +52,3 (C = 0,44 in Chloroform)

Das Protonenspektrum und die Massenbestimmung liefert identische Ergebnisse zum Racemat.

Ausgehend von 379 mg (1,0 mmol) des (+)-Enantiomers (7b) erhält man 377 mg des entsprechenden Tosylats.

Fp° C: 157,5 - 158° C

$[\alpha]_D^{17}$ = -51,2 ( C = 0,32 in Chloroform)

Die Spektren ($^1$H-NMR und IR) sind identisch mit denen des oben aufgeführten (-)-Enantiomers (7c).

Beispiel 9

(+)-1-methyl-3-hydroxy-4-p-chloro-phenyl-α-(p-tosyloxybenzyl)-pyrrolidin-2-on

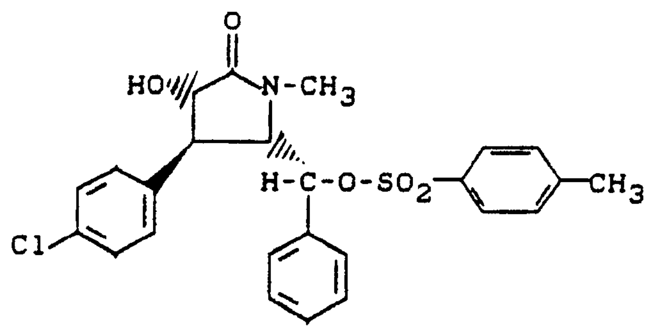

Einer Lösung von 1,24 g (+)-1-Methyl-3-tetrahydropyranyloxy-4-p-chlorophenyl-5-benzoyl-pyrrolidin-2-on (6b) in 20 ml wasserfreiem Tetrahydrofuran wird bei -25° C bis -30° C langsam unter Rühren zu einer vorgekühlten 1,0 M Lithium-tri-sec-butyl-borhydrid-Lösung in Tetrahydrofuran zugetropft. Es wird anschließend noch 2 h gerührt. Danach werden bei -25° C - -30° C 860 mg Tosylchlorid in 4 ml Tetrahydrofuran zugegeben, eine halbe Stunde bei dieser Temperatur und dann 2 h bei Raumtemperatur gerührt. Die Reaktionslösung wird über Nacht stehen gelassen. Die Reaktionslösung wird auf 35 ml Eiswaseer gegeben und 3 mal mit je 25 ml Benzol extrahiert. Die gesammelten organischen Phasen werden mit verdünnter Salzsäure, verdunnter Natriumhydroxidlösung und Natriumbicarbonatlösung gewaschen und über Natriumsulfat getrocknet. Es wird vom Rückstand abfiltriert und das Filtrat bis fast zur Trockene eingeengt. Zum verbleibenden Rückstand werden 15 ml wasserfreies Ethanol und 50 mg p-Toluolsulfonsäure zugegeben. Die Reaktionslöung wird 25 min auf 60° C erhitzt. Nach Filtration erhält man weiße Nadeln, die mit Ether gewaschen werden.

Ausbeute: 1,3 g (89% der Theorie)

Fp° C: 179-180° C

$^1$H-NMR ($CDCl_3$, 90 MHz): δ = 1,93 (1H, OH); 2,37 (s, 3H, Ar-$CH_3$); 2,84 (s, 3H, $NCH_3$); 3,18 (t, 1H, J = 6 Hz, $C_4$-H); 3,83 (dd, 1H, J = 4 Hz, $C_5$-H); 4,09 (d, 1H, J = 6 Hz, $C_3$-H); 5,70 (d, 1H, J = 4 Hz, $C_7$-H); 6,6 - 7,6 (m, 13H, ArH).

Beispiel 10

(±)-1-Methyl-3-hydroxy-4-p-fluorophenyl-5-α-(p-tosyloxybenzyl)pyrrolidin-2-on

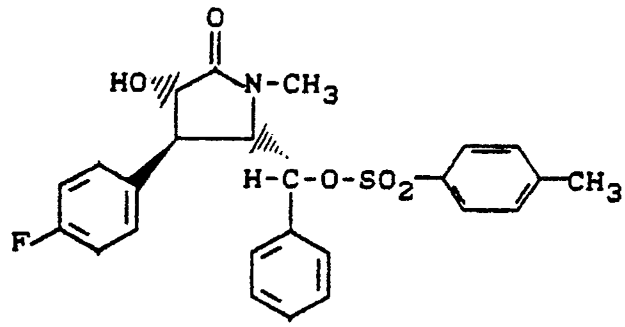

In Analogie zu der Vorschrift des Beispiels 9 wird die Titelverbindung aus 1,19 g der Verbindung aus Beispiel 6a erhalten.
Ausbeute: 1,19 g (84,3% der Theorie)
Fp° C: 162-164° C

Beispiel 11a,b,c

(±) 11a; (+) 11b; (-) 11c

Dehydrocycloclausenamid

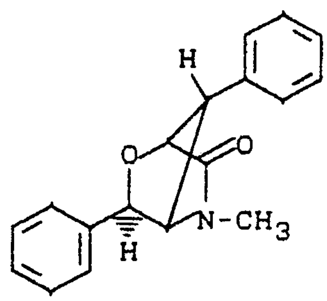

a) Eine Lösung von 1,35 g (3 mmol) der Verbindung aus Beispiel 8a, 8b oder 8c in 100 ml 2,6-Lutidin wird unter Stickstoffatmosphäre über 5 h unter Rühren leicht erhitzt. Anschließend werden 170 ml Methylenchlorid hinzugefügt. Die Reaktionslösung wird nacheinander mit 30 ml Wasser und 3 mal mit je 40 ml 6 N Salzsäure, Wasser, gesättigter Natriumcarbonat- und gesättigter Natriumchloridlösung gewaschen. Nach Trocknung über wasserfreiem Natriumsulfat und Abfiltrieren wird das Lösemittel entfernt und 0,914 g eines braunen öligen Rückstandes fällt an. Dieser verfestigt sich beim Stehenlassen und wird aus 4 ml Methanol kristallisiert. Der weiße Niederschlag hat einen Schmelzpunkt von 158 - 159° C. Nach Chromatographie auf einer mit 30 g $SiO_2$ beschichteten Säule werden 450 mg kristallines Produkt vom Schmelzpunkt 164-166° C gewonnen. Dieser Schmelzpunkt ist mit dem des (-)-Dehydrocycloclausenamids identisch. Aus dem Filtrat der Chromatographie können weitere 100 mg der Titelverbindung gewonnen werden.
Ausbeute ges.: 550 mg (66% der Theorie)
Summenformel: $C_{18}H_{17}NO_2$

| | | | | |
|---|---|---|---|---|
| Elementaranalyse: | kal.: | C 77,39 | H 6,14 | N 5,02 |
| | gef. : | C 77,30 | H 5,84 | N 4,92 |

$^1H$-NMR ($CDCl_3$, 300 MHz): δ = 2,96 (s, 3H, $NCH_3$); 3,615 (s, br, 1H, $C_4$-H); 4,101 (1H, $C_5$-H); 4,835 (dd, 1H, J = 1,2 Hz, 2,7 Hz, $C_3$-H); 5,014 (s, 1H, $C_7$-H); 7,0 - 7,5 (m, 10H, ArH).

b) Eine Lösung von 0,174 g (1 mmol) Diethylazodicarbonsäure in 3 ml Tetrahydrofuran wird tropfenweise innerhalb von 2 h bei Raumtemperatur zu einer Lösung von Neoclausenamid (0,297 g, 1 mmol) und 0,262 g (1 mmol) Triphenylphosphan in 6 ml Tetrahydrofuran zugetropft. Es wird 24 h gerührt und anschließend vom weißen Niederschlag abfiltriert. Das Filtrat wird im Vakuum eingeengt und der verbleibende Rückstand durch präparative Dünnschichtchromatographie im Laufmittelsystem Chloroform/Methanol (9:1) gereinigt. Man erhält 87 mg (31% der Theorie) der Titelverbindung. Die $R_f$-Werte und die IR-, $^1H$-NMR-und Massenspektroskopiedaten sind identisch mit dem natürlich gewonnenen Produkt. Die spezifische Drehung mit + 0,00 der synthetisch hergestellten Verbindung weist diese als Racemat aus.

$^1H$-NMR ($CDCl_3$, 90 MHz): δ = 2,95 (s, 3H, $NCH_3$); 3,60 (s, 1H, $C_4$-H); 4,09 (s, 1H, $C_5$-H); 4,81 (s 1H, $C_3$-H); 5,00 (s, 1H, $C_7$-H); 7,1 -7,5 (m, 10H, ArH).

Optisch aktive Isomere:

Ausgehend von 640 mg der Verbindung aus Beispiel 8b werden 255 mg (64,4% der Theorie) des Beispiels 11b nach Umkristallisation aus Methanol und Chromatographie der Mutterlauge gewonnen.

Fp °C = 199 - 199,5 °C

$[\alpha]_D^{15}$ = -88,3 (C = 0,14 in Methanol)

Das natürlich gewonnene Produkt hat zum Vergleich den Schmelzpunkt: 164 - 166 °C

$[\alpha]_D^{15}$ = - 40,0 (c = 0,23 in Methanol)

$^1H$-NMR ($CDCl_3$, 90 MHz): δ = 2,94 (s, 1H, $NCH_3$); 3,58 (s, 1H, $C_4$-H); 4,05 (s, 1H, $C_5$-H); 4,77 (m, 1H, $C_3$-H); 4,97 (s, 1H, $C_7$-H); 6,98 -7,48 (m, 10H, ArH).

Ausgehend von 225 mg der Verbindung aus Beispiel 8c erhält man 77 mg (55% der Theorie) des (-)-Enantiomers 11c nach Umkristallisation.

Fp °C: 204 - 204,5 °C

$[\alpha]_D^{18}$ = +86,2 (C =0,13 in Methanol)

$^1H$-NMR ($CDCl_3$, 90 MHz): δ = 2,93 (s, 3H); $NCH_3$); 3,56 (s, 1H, $C_4$-H); 4,04 (s, 1H, $C_5$-H); 4,86 (m, 1H, $C_3$-H); 4,96 (s, 1H, $C_7$-H); 6,97 - 7,44 (m, 10H, ArH).

Beispiel 12

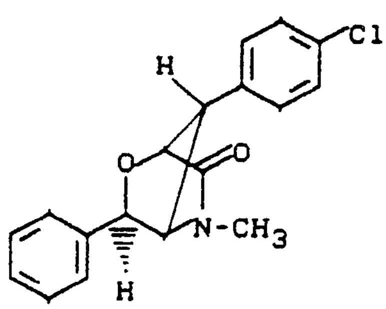

0,97 g (2,0 mmol) 1-Methyl-3-hydroxy-4-p-chlorophenyl-5-(α-tosyloxybenzyl)-pyrrolidin-2-on (8a) werden in 75 ml 2,6-Lutidin gelöst. Es wird 5 h bei 145 °C gerührt. Danach wird Lutidin abdestilliert und der verbleibende Rückstand in 60 ml Chloroform aufgenommen und nacheinander 2 mal mit 10 ml 4 N Salzsäure, 20 ml Wasser, gesättigter Natriumcarbonat- und Natriumchloridlösung gewaschen und über wasser freiem Natriumsulfat getrocknet. Nach Abfiltrieren und Entfernen des Lösemittels erhält man einen braunen öligen Rückstand, der auf einer mit 40 g $SiO_2$ beschichteten Säule in dem Laufmittelsystem Chloroform/Aceton (100:3) eluiert wird. Man erhält 430 mg eines Feststoffs, dessen dünnschichtchromatographische Untersuchung zwei $R_f$-Werte 0,64 und 0,56 ergibt. Der Feststoff wird mit Isopropanol umkristallisiert und ergibt ein kristallines Produkt von 337 mg (53% der Theorie) mit dem Schmelzpunkt 158-160 °C und dem $R_f$-Wert = 0,56.

$^1$H-NMR ($CDCl_3$, 90 MHz): $\delta$ = 2,94 (s, 3H, $NCH_3$); 3,53 (s, 1H, $C_4$-H); 4,03 (s, 1H, $C_5$-H); 4,76 (m, 1H, $C_3$-H); 4,98 (s, 1H, $C_7$-H); 6,94 - 7,46 (m, 9H, ArH).

Beispiel 13

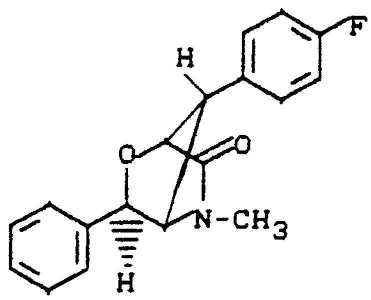

Ausgehend von 944 mg (2,01 mmol) der Verbindung aus Beispiel 8a wird die Titelverbindung in Analogie zu der Vorschrift für Beispiel 12 hergestellt.
Ausbeute: 342 mg (57,7% der Theorie)
Fp° C: 163-164° C
$R_f$ = 0,51 ($SiO_2$-Platte, Chloroform/Aceton 100:3)
$^1$H-NMR ($CDCl_3$, 90 MHz): $\delta$ = 2,95 (s, 3H, $NCH_3$); 3,54 (s, 1H, $C_4$-H); 4,03 (s, 1H, $C_5$-H); 4,76 (s, 1H, $C_3$-H); 4,98 (s, 1H, $C_7$-H); 6,80 - 7,50 (m, 9H, ArH).

**Ansprüche**

1. Verfahren zur Herstellung der Verbindungen der allgemeinen Formel

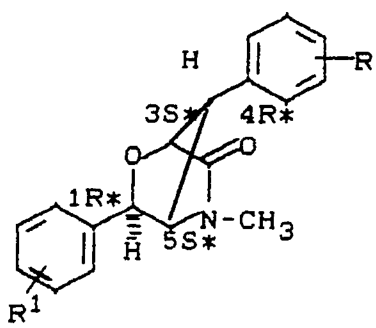

(I)

in welcher
R und $R^1$ für Wasserstoff oder Halogen stehen,
als Racemat (±), als (+)- oder als (-)-Enantiomere, dadurch gekennzeichnet, daß man
[A] Verbindungen der allgemeinen Formel (II)

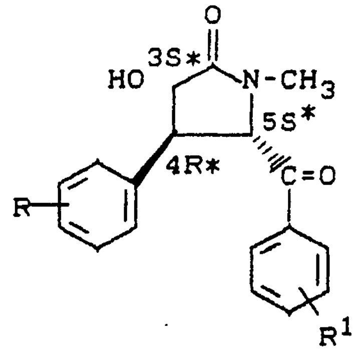

(±), (+), (-) (II)
(R,S-Nomenklatur wird beispielhaft an den Verbindungen der allgemeinen Formel (II) aufgeführt)

in welcher
R und $R^1$ für Wasserstoff oder Halogen stehen,
entweder als Racemat (±) oder als entsprechendes (+)-oder (-)-Enantiomer, zunächst mit 2,3-Dihydropyran zu Verbindungen der allgemeinen Formel (III),

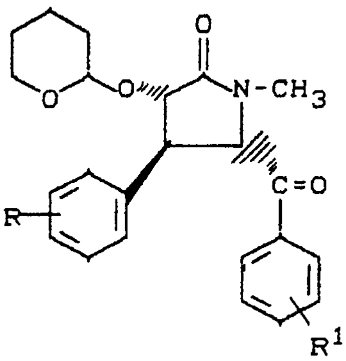

(±), (+), (-) (III)

in welcher
R und $R^1$ die oben angegebene Bedeutung haben,
verethert, dann nach üblicher Methode zu Verbindungen der allgemeinen Formel (IV),

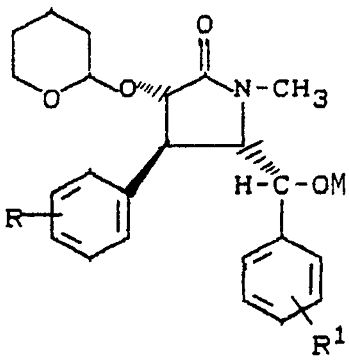

(±), (+), (-) (IV)

in welcher
R und $R^1$ die oben angegebene Bedeutung haben,
reduziert, dann in einem weiteren Schritt mit p-Methyltoluolsulfonsäurechlorid der Formel (V)

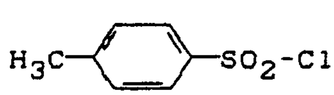

(V) .

zu Verbindungen der allgemeinen Formel (VI)

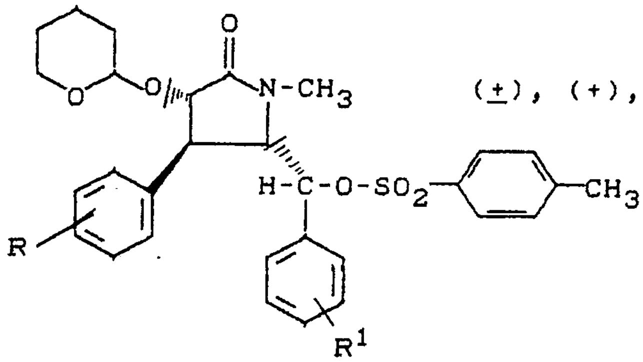

(±), (+), (-) (VI)

in welcher

R und $R^1$ die oben angegebene Bedeutung haben,

umsetzt, dann die Tetrahydropyranylschutzgruppe nach üblicher Methode abspaltet und in einem letzten Schritt mit 2,6-Lutidin der Formel

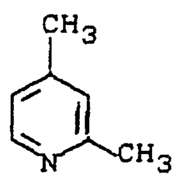

(VII)

umsetzt,

oder daß man

[B] Verbindungen der allgemeinen Formel (IVa)

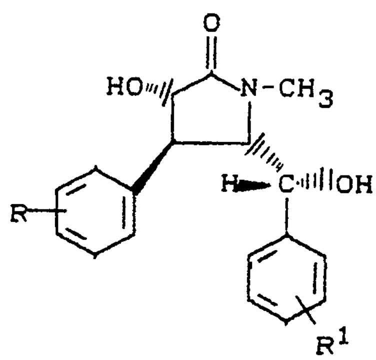

(±) (IVa)

in welcher

R und $R^1$ die oben angegebene Bedeutung haben,

in der racemischen Form (±) mit Azodicarbonsäurediethylester der Formel (VIII),

$$\begin{matrix} N-CO_2-C_2H_5 \\ \| \\ N-CO_2-C_2H_5 \end{matrix} \quad (VIII)$$

gegebenenfalls in Anwesenheit eines Hilfsstoffes, in inerten Lösemitteln umsetzt.

2. Verfahren nach Anspruch 1, dadurch gekennzeichnet, daß man die Veretherung in einem Temperaturbereich von -10 °C bis +50 °C durchführt.

3. Verfahren nach Anspruch 1 und 2, dadurch gekennzeichnet, daß man die Reduktion der Hydroxygruppe mit Metallhydriden und komplexen Metallhydriden wie beispielsweise Lithiumboranate, Natriumboranate, Lithiumaluminiumhydrid, Zinkborhydride oder Aluminiumtriisopropylat als Reduktionsmittel durchführt.

4. Verfahren nach Ansprüchen 1 bis 3, dadurch gekennzeichnet, daß man zur Abspaltung der Tetrahydropyranyl-Schutzgruppe Protonensäuren einsetzt.

5. Verfahren nach Ansprüchen 1 bis 4, dadurch gekennzeichnet, daß man zur Umsetzung mit Azodicarbonsäureester als Hilfsstoffe Trialkylphosphane wie beispielsweise Triphenylphosphan oder Ethyldiphenylphosphan, bevorzugt Triphenylphosphan, einsetzt.

6. Verfahren zur Herstellung der Verbindungen der Formel (II),

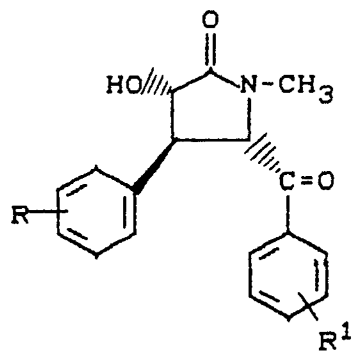

(±) (II)

in welcher

R und $R^1$ für Wasserstoff oder Halogen stehen,

in Form ihrer reinen (+)- bzw (-)-Enantiomeren, dadurch gekennzeichnet, daß man Verbindungen der Formel (II) in ihrer Racem-Form (±) durch die Umsetzung mit (-)-Menthoxyessigsäurechlorid der allgemeinen Formel (X),

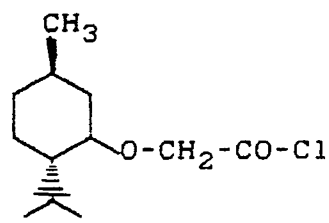

(X)

in einem inerten Lösemittel, vorzugsweise Methylenchlorid in Anwesenheit einer Base die entsprechenden Diastereomere herstellt und anschließend mit Säuren den Menthoxyessigsäurerest unter Freisetzung der Hydroxylfunktion abspaltet.

7. Verfahren nach Anspruch 6, dadurch gekennzeichnet, daß man die Umsetzung in einem Temperaturbereich +50 °C bis +150 °C durchführt.

8. Verbindungen der allgemeinen Formel (Ib)

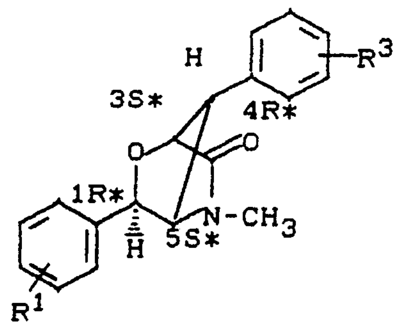

(Ib)

in welcher
$R^3$ für Halogen steht und
$R^1$ für Wasserstoff oder Halogen steht,
als Racemat (±) und als (+)- oder (-)-Enantiomere.

9. Arzneimittel enthaltend eine Verbindung nach Anspruch 8.

10. Verwendung der Verbindungen nach Anspruch 8 zur Herstellung von Arzneimitteln.